# EUROPEAN PATENT SPECIFICATION

(11) **EP 1 037 616 B1**
(45) Date of publication and mention of the grant of the patent: **01.03.2006**
(21) Application number: 98947741.9
(22) Date of filing: 29.10.1998
(51) Int. Cl.: A61K 31/495

(54) **COMBINATION OF ALPHA-1-ADRENERGIC ANTAGONISTS AND A CGMP PDEv INHIBITOR FOR THE TREATMENT OF IMPOTENCE**
KOMBINATION AUS EINEM ALPHA-1-ADRENOREZEPTOR ANTAGONISTEN UND EINEM CGM PDEv HEMMER ZUR BEHANDLUNG VON IMPOTENZ
COMPOSITION D'UN INHIBITEUR PDE ET D'UN ANTAGONISTE ALPHA-ADRENERGIQUE POUR LE TRAITEMENT DE L'IMPUISSANCE

(30) Priority: 16.12.1997 US 69741 P
(43) Date of publication of application: 27.09.2000
(73) Proprietor: Pfizer Products Inc., Groton, Connecticut 06340 (US)
(72) Inventor: WYLLIE, Michael, Grant, Canterbury Kent CT1 3DS (GB)
(74) Representative: McMunn, Watson Palmer
(86) International application number: PCT/IB1998/001723
(87) International publication number: WO 1999/030697

(56) References cited:
- EP-A- 0 268 146
- EP-A- 0 459 666
- WO-A-97/18814
- US-A- 5 145 852
- US-A- 5 474 535
- "WPI WORLD PATENT INFORMATION DERWENT" WPI WORLD PATENT INFORMATION DERWENT, vol. 27, no. 97, XP002095495
- KITZEN ET AL: "Potentiation of Phosphodiesterase Inhibitor Antithrombotic Activity with Alpha-2 Adrenergic Blockade" LIFE SCIENCES, vol. 7, no. 48, 1991, page PL31 PL31 XP002076709
- HEDLUND ET AL: "Pharmacotherapy in Erectile Dysfunction Agents for Self-Injection Programs and Alternative Application Models" SCANDINAVIAN JOURNAL OF UROLOGY AND NEPHROLOGY, vol. S179, no. 30, 1996, page 129 129 XP002076708
- BENET ET AL: "The Medical Treatment of erectile Dysfunction" DRUGS OF TODAY, vol. 32, no. 6, September 1996, pages 483-499, XP002095494

## Description

### Field Of The Invention

This invention relates to the treatment of impotence comprising co- administering (1) an α₁-adrenergic receptor antagonist and (2) an agent which is a cyclic guanosine 3',5'-monophosphate (cGMP) PDEᵥ inhibitor. The combination is particularly suitable for the treatment of patients suffering from impotence or erectile dysfunction.

### Background Of The Invention

Impotence is the inability to obtain and/or sustain an erection sufficient for penetration of the vagina and/or intercourse. Thus, impotence is also referred to as "erectile insufficiency" or "erectile dysfunction". It has been estimated that 10-12 million American men between the ages of 18 and 75 suffer from chronic impotence, with the great majority being over age 55.

The penis normally becomes erect when certain tissues, in particular the corpora cavernosa in the central portion of the penis, become engorged with blood, thereby causing them to become less flaccid, and in turn causing an erection.

Impotence can result from psychologic disturbances (psychogenic), from physiologic abnormalities (organic) or from a combination of both. Thus, in some males erectile dysfunction may be due to anxiety or depression, with no apparent somatic or organic impairment. In other cases, erectile dysfunction is associated with atherosclerosis of the arteries supplying blood to the penis. In still other cases, the dysfunction may be due to venous leakage or abnormal drainage in which there is leakage from veins in the penis such that sufficient pressure for an erection can be neither obtained nor maintained. In still other cases, the dysfunction is associated with a neuropathy or due to nerve damage arising from, for example, surgery or a pelvic injury. Typically, multiple factors are responsible for impotence.

α-Adrenergic receptors (herein also referred to as "α-adrenoceptors" or as "α-receptors") are specific protein recognition sites located in the peripheral and central nervous systems and other tissues throughout the body. Neurotransmitters such as norepinephrine control many physiologic functions via an action on these receptors and thereby transmit information between cells or influence biochemical processes within the cell. Many agents capable of modifying norepinephrine activity on α-adrenoceptors have been developed over the last 40 years.

Drugs active at α-adrenoceptors can be broken into two major classes, agonists and antagonists. Agonists, of which clonidine and naphazoline are examples, activate the receptor system in the same way as the endogenous neurotransmitters, norepinephrine and epinephrine. Antagonists, of which phenoxybenzamine and prazosin are examples, do not activate the receptor but block the actions of the endogenous neurotransmitters.

Different α-adrenoceptor types have been discovered over the years including α₁-adrenoceptors and α₂-adrenoceptors. These receptor types are now considered to be further subdivided into subtypes including 1A, 1B, 1D, 1H, 1L, 1N, 2A, 2B, and 2C.

α₂-Adrenoceptors located on nerve terminals, by an action dependent at least in part on neurotransmitter release, are known to reduce activity in the sympathetic nervous system and increase activity within the parasympathetic nervous system, particularly in the vagus nerve. In addition, α₂-adrenoceptors on other tissues in the body control platelet aggregation, lipolysis and metabolism. α₂-Adrenoceptor antagonists have been disclosed for a wide variety of therapies, including reversing the state of anesthesia (US 5,636,204), for the treatment of glaucoma (US 4,590,202), for the treatment of cognitive disorders such as endogenous depression, age dependent memory impairment, and Alzheimer's disease (US 5,498,623), and for the treatment of numerous other neurodegenerative disorders (US 5,281,607).

α₁-Adrenoceptors are known to mediate the contraction of arterial and venous smooth muscle. α₁-Adrenoceptor antagonists have been used widely as first line therapy for the treatment of hypertension and, more recently, for the symptomatic relief of benign prostatic hyperplasia, BPH. See Kenny et al., Exp. Opin. Invest. Drugs (1995) 4(10), pp 915-923. Some compounds which have α₁-adrenoceptor antagonist activity, such as phentolamine and trazodone are used to treat impotence, although the mechanism (or mechanisms) of promoting erectile function is not completely understood. Such compounds are believed to work at least in part through blocking the action of norepinephrine which, without being blocked, otherwise causes contraction of the cavernosal smooth muscle allowing venous blood to leave the penis, and thereby produces de-tumescence and flaccidity of the organ. Many such compounds have been delivered locally by intra-cavernosal injection and are often associated with complications such as priapism (prolonged and painful erection), pain and infection at the site of injection and, in the long term, tissue fibrosis. Apart from the obvious discomfort, there is an associated loss of spontaneity.

α-Adrenoceptors can also mediate a reduction in cavernosal smooth muscle contraction indirectly by reducing sympathetic nervous activity by central actions, such effect being known for trazadone, and certain centrally active α₂-receptor agonists such as clonidine, or by a direct action on the smooth muscle cells as exemplified by papaverine.

Agents which elevate cGMP levels are also well known and can work through any of several mechanisms. Agents which selectively inhibit an enzyme predominantly involved in cGMP breakdown, for example a cGMP phosphodiesterase (cGMP PDE), constitute one example. Other phosphodiesterases can also hydrolyze cGMP, and inhibitors of these enzymes including compounds such as rolipram, zaprinast and xanthine derivatives such as caffeine, theophylline and theobromine, can accordingly influence cGMP levels. Other compounds which increase cGMP levels can do so through different mechanisms including the activation of soluble guanylate cyclase or membrane-bound guanylate cyclase, either directly as in the case of atrial natriuretic peptide, or indirectly. Other compounds act to increase cellular cGMP levels by modulation of cytokines. Other classes of cGMP elevators include muscarinic agonists, which can elevate cGMP levels without altering phosphodiesterase activity. Some prostaglandins such as PGE₁ are also known cGMP elevators. Kanba et. al., J. Neurochem., Vol. 57, No. 6, 1991.

Cyclic guanosine 3',5'-monophosphate phosphodiesterase (cGMP PDE) inhibitors are widely known as cardiovascular agents for the treatment of conditions such as angina, hypertension, and congestive heart failure. More recently cGMP PDE inhibitors have been found to be effective for the treatment of impotence, importantly by oral administration. See, for example, PCT/EP94/01580, published as WO 94/28902. It is believed that such compounds may manifest their therapeutic effects by achieving high cGMP levels through inhibiting phosphodiesterase, thereby relaxing and expanding cavernosal cells and blocking the outflow of blood from the penis.

### Summary of the Invention

This invention provides for the use of:
(1) a compound selected from α₁-adrenoceptor antagonists (herein also referred to as α₁-antagonists), and
(2) a compound which is a cGMP PDEᵥ inhibitor for the manufacture of a medicament for treating impotence (also known in the art and referred to herein as "male erectile dysfunction"), especially in humans.

Reference to a compound or agent within the scope of (1) or (2), above, such as to an a₁-antagonist and/or to a cGMP PDE_{V} inhibitor, both in this disclosure and the appendant claims, shall at all times be understood to include all active forms of such agents, including the free form thereof (e.g., the free acid or base form) and also all pharmaceutically acceptable salts, prodrugs, polymorphs, hydrates, solvates, stereoisomers (e.g. diastereomers and enantiomers), and so forth. Active metabolites of either the α₁-antagonist or the cGMP PDEᵥ inhibitor, in any form, are also induded.

In the context of the known α₁-adrenoceptor subtypes, antagonists at 1A, 1B, 1D, 1H, 1 N and IL are equally preferred.

cGMP PDE inhibitors which are selective inhibitors of the cGMP PDEv isoenzyme are disclosed in US patents 5,250,534, 5, 346,901, 5,272,147, and in the international patent application published as WO 94/28902 designating, *inter alia,* the U.S.

Preferred combinations of an α₁-adrenoceptor antagonist and a cGMP PDEᵥ inhibitor useful herein are "synergistic", meaning that the therapeutic effect of co-administering compounds selected from (1) and (2) as defined above is greater than additive. Thus, co-administering both therapeutic agents produces an effect which is greater than the sum of the effects of each agent administered alone. Such synergy is advantageous in that it allows for each therapeutic agent typically to be administered in an amount less than if the combined therapeutic effects were additive. Thus, therapy can be effected for patients who, for example, do not respond adequately to the use of one component at what would be considered a maximal strength dose. Additionally, by administering the components in lower amounts relative to the case where the combined effects are additive, side effects such as priapism or pain at the site of injection can be minimized or avoided in many cases. Such synergy can be demonstrated by the tests disclosed below.

Additional preferred combinations include those which can be taken "on demand", as opposed to needing to be taken chronically. Such preferred combinations include those which modulate the sexual response such that the patient responds to sexual (e.g., visual) stimulation, as opposed to compositions which act by causing an erection in the absence of sexual stimulation.

Additional preferred combinations include those which are "fast acting", meaning that the time taken from administration to the point at which the sexual response can be modulated is less than about two hours, preferably less than about one hour, more preferably on the order of a half hour or less, and even more preferably within 10 or 15 minutes.

" Co-administration" when used in this disclosure and the appendant claims, for example in referring to a combination of an α₁-antagonist and a cGMP PDEᵥ inhibitor, means that the individual components can be administered together as a composition if the route of administration for each component is the same. Thus the invention further provides a composition comprising
(1) a first compound, said first compound being selected from α₁- adrenoceptor antagonists;
(2) a second compound which is a cGMP PDEᵥ inhibitor; and
(3) a pharmaceutically acceptable carrier.

A preferred group of compositions are synergistic.

"Co-administration" also includes administering each of compounds (1) and (2) separately but as part of the same therapeutic treatment program or regimen, and it is contemplated that separate administration of each compound, at different times and by different routes, will sometimes be recommended. Thus, the two compounds need not necessarily be administered at essentially the same time. In a preferred embodiment, administration is timed so that the peak pharmacokinetic effect of one compound coincides with the peak pharmacokinetic effect for the other. If co- administered separately, it is also preferred that both of compounds (1) and (2) be administered in an oral dosage form.

Reference herein to a "combination" is to the co-administration of a compound selected from (1) and a compound selected from (2), either as a composition or separately, e.g., by different routes of administration.

Since the present invention has an aspect that relates to the treatment of impotence by treatment with a combination of compounds which may be co-administered separately, the invention also relates to combining separate pharmaceutical compositions in kit form. The kit comprises two separate pharmaceutical compositions: (1) a composition comprising a compound selected from α₁-adrenergic receptor antagonists, plus a pharmaceutically acceptable carrier or diluent; and (2) a composition comprising a compound selected from agents which are cGMP PDEᵥ inhibitors, plus a pharmaceutically acceptable carrier or diluent. The amounts of (1) and (2) are such that, when co-administered separately, the impotence condition is treated and/or remediated. The kit comprises a container for containing the separate compositions such as a divided bottle or a divided foil packet, wherein each compartment contains a plurality of dosage forms (e.g., tablets) comprising (1) or (2). Alternatively, rather than separating the active ingredient- containing dosage forms, the kit may contain separate compartments each of which contains a whole dosage which in turn comprises separate dosage forms. An example of this type of kit is a blister pack wherein each individual blister contains two (or more) tablets, one (or more) tablet(s) comprising pharmaceutical composition (1), and the second (or more) tablet(s) comprising pharmaceutical composition (2). Typically the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician. In the case of the instant invention a kit therefore comprises
(1) a therapeutically effective amount of a composition comprising a compound selected from α₁-adrenergic receptor antagonists, plus a pharmaceutically acceptable carrier or diluent, in a first dosage form;
(2) a therapeutically effective amount of a composition comprising a compound selected from compounds which are cGMP PDEᵥ inhibitors, plus a pharmaceutically acceptable carrier or diluent, in a second dosage form; and
(3) a container for containing said first and second dosage forms.

An example of such a kit, alluded to above, is a so-called blister pack. Blister packs are well known in the packaging industry and are widely used for the packaging of pharmaceutical unit dosage forms such as tablets, capsules, and the like. Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably, the strength of the sheet is such that the tablets or capsules can be removed from the blister pack by manually applying pressure on the recesses whereby an opening is formed in the sheet at the place of the recess. Tablet(s) or capsule(s) can then be removed via said opening.

It may be desirable to provide a memory aid on the kit, e.g., in the form of numbers next to the tablets or capsules whereby the numbers correspond with the days of the regimen during which the tablets or capsules so specified should be ingested. Another example of such a memory aid is a calendar printed on the card, e.g., as follows "First Week, Monday, Tuesday, ...etc.... Second Week, Monday, Tuesday etc. Other vahations of memory aids will be readily apparent. A "daily dose" can be a single tablet or capsule or several pills or capsules to be taken on a given day. Also a daily dose of the first compound can consist of one tablet or capsule while a daily dose of the second compound can consist of several tablets or capsules and vice versa. The memory aid should reflect this.

Other pharmaceutical components may also be optionally included as part of the combinations useful in this invention so long as they do not interfere or adversely affect the effects of the α₁-antagonist/cGMP PDEᵥ inhibitor combination.

Compounds selective for the PDEv isoenzyme are disclosed and characterized, for example, in PCT/EP94/01580, published as WO 94/28902 and which designates, *inter alia,* the United States.

Preferred cGMP PDEᵥ inhibitors include sildenafil which has the structure: 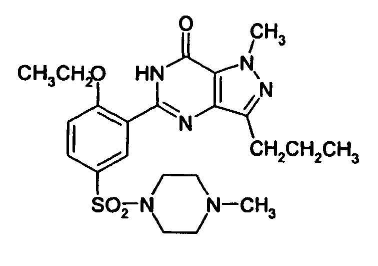 and pharmaceutically acceptable salts thereof, and the compound having the structure: 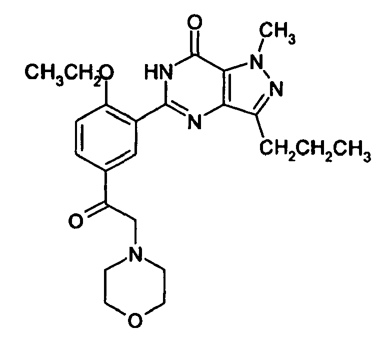 and pharmaceutically acceptable salts thereof. The second compound is disclosed, for example, in US patents 5, 272,147 and 5,426,107.

A preferred pharmaceutically acceptable salt of sildenafil for use in this invention is the citrate salt, disclosed in co-pending U. S. Application No. 081944,546 filed October 7, 1997.

Also preferred are compounds disclosed in PCT/EP95/00183, published as WO 95/19978 designating, *inter alia,* the United States said compounds having the formula 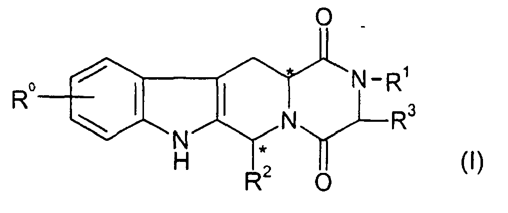 and salts and solvates thereof, in which:
R⁰ represents hydrogen, halogen or C₁-₆alkyl,;
R¹ represents hydrogen, C₁-₆alkyl, C₂-₆alkenyl, C₂-₆alkynyl, haloC₁-₆alkyl, C₃₋₈cycloalkyl, C₃-₈cycloalkylC₁-₃alkyl, arylC₁-₃alkyl or heteroarylC₁-₃alkyl;
R² represents an optionally substituted monocyclic aromatic ring selected from benzene, thiophene, furan and pyridine or an optionally substituted bicyclic ring 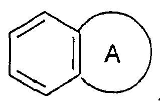 attached to the rest of the molecule via one of the benzene ring carbon atoms and wherein the fused ring A is a 5- or 6-membered ring which may be saturated or partially or fully unsaturated and comprises carbon atoms and optionally one or two heteroatoms selected from oxygen, sulphur and nitrogen; and
R³ represents hydrogen or C₁₋₃alkyl, or R¹ and R³ together represent a 3- or 4-membered alkyl or alkenyl chain.

A preferred subset of compounds having formula la (also disclosed in WO 95/19978) includes compounds of the formula 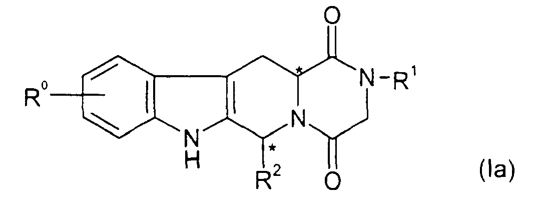
and salts and solvates thereof, in which:
R⁰ represents hydrogen, halogen or C₁-₆alkyl;
R¹ represents hydrogen, C₁₋₆alkyl, haloC₁₋₆alkyl, C₃₋₈cycloalkyl, C₃₋₈cycloalkyl-C₁₋₃alkyl, arylC₁₋₃alkyl or heteroarylC₁₋₃alkyl; and
R² represents an optionally substituted monocyclic aromatic ring selected from benzene, thiophene, furan and pyridine or an optionally substituted bicyclic ring 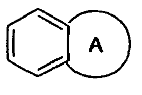 attached to the rest of the molecule via one of the benzene ring carbon atoms and wherein the fused ring A is a 5- or 6- membered ring which may be saturated or partially or fully unsaturated and comprises carbon atoms and optionally one or two heteroatoms selected from oxygen, sulphur and nitrogen.

A specific compound within formulae (1) is:
(6R, 12aR)-2,3,6,7,12,12a-hexahydro-2-methyl-6-(3,4- methylenedioxyphenyl) - pyrazino[2',I':6,1]pyrido[3,4-b]indole-1,4-dione.

Preferred α-antagonists include doxazosin, terazosin, abanoquil, and prazosin, and the pharmaceutically acceptable salts thereof (especially doxazosin mesylate, terazosin hydrochloride, and prazosin hydrochloride), which are selective for α₁ adrenoceptors. Preferred specific combinations include any of these in combination with sildenafil or a pharmaceutically acceptable salt thereof, particularly the citrate salt. Most preferred are sildenafil citrate in combination with doxazosin mesylate or abanoquil mesylate.

Examples of additional α-antagonists include alfuzosin, indoramin, naftopidil, tamsulosin, dapiprazole, and also pharmaceutically acceptable salts thereof. The specific compounds above are reported to be selective for α₁ receptors.

Further α-antagonists which are reported to be specific for α₁ include:
Recordati 15/2739 which has the structure 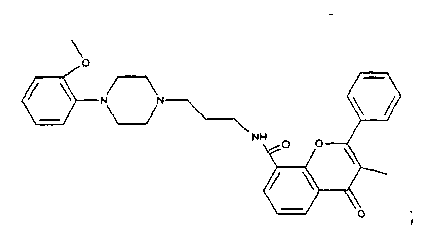
SNAP 1069 which has the structure 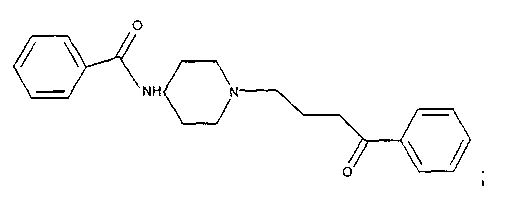
SNAP 5089 which has the structure 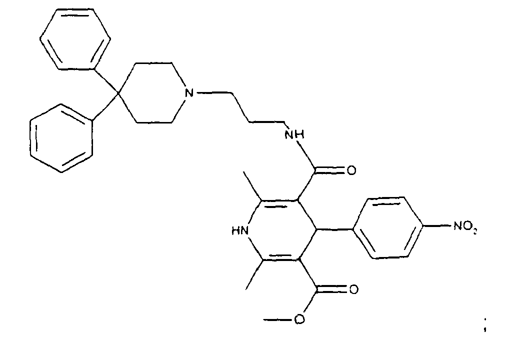
RS 17053 which has the structure 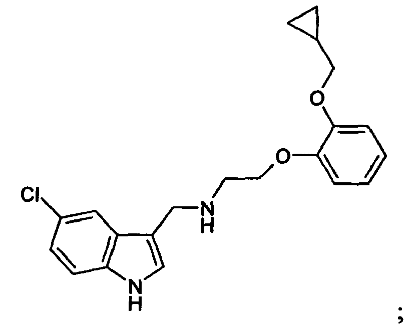 and
SL 89.0591 which has the structure

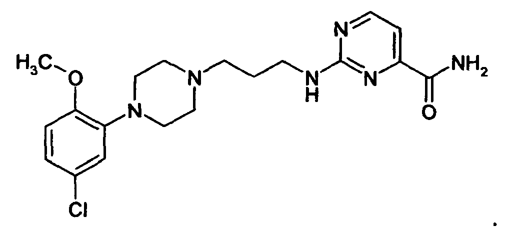

Combinations of sildenafil, especially sildenafil citrate, with an α₁- selective antagonist, including any of those previously noted, are preferred.

### Detailed Description

The cGMP PDE inhibitors useful in this invention as cGMP elevators may be widely chosen from among any of those already known to the art or subsequently discovered and/or hereafter developed. Suitable cGMP PDE inhibitors include those disclosed in any of the following US patents:
a 5-substituted pyrazolo[4,3-d]pyrimidine-7-one as disclosed in US 4,666,908;
a griseolic acid derivative as disclosed in any of US 4,634,706, 4,783,532, 5,498,819, 5,532,369, 5,556,975, and 5,616,600;
a 2- phenylpurinone derivative as disclosed in US 4,885,301;
a phenylpyridone derivative as disclosed in US 5,254,571;
a fused pyrimidine derivative as disclosed in US 5,047,404;
a condensed pyrimidine derivative as disclosed in US 5,075,310;
a pyrimidopyrimidine derivative as disclosed in US 5,162,316;
a purine compound as disclosed in US 5,073,559;
a quinazoline derivative as disclosed in US 5,147,875;
a phenylpyrimidone derivative as disclosed in US 5,118,686;
an imidazoquinoxalinone derivative or its aza analog as disclosed in US 5,055,465 and 5,166,344;
a phenylpyrimidone derivative as disclosed in US 5,290,933;
a 4-aminoquinazoline derivative as disclosed in US 5,436,233 or 5,439,895;
a 4,5-dihydro-4-oxo-pyrrolo[1,2-a]quinoxaline derivative as disclosed in US 5,405,847;
a polycyclic guanine derivative as disclosed in US 5,393,755;
a nitogenous heterocyclic compound as disclosed in US 5,576,322;
a quinazoline derivative as disclosed in US 4,060,615; and
a 6-heterocyclyl pyrazolo[3,4-d]pyrimidin-4-one as disclosed in US 5,294,612.

Other disclosures of cGMP PDE inhibitors include the following, all of which are herein incorporated by reference:
European patent Application (EPA) publication no. 0428268;
European patent 0442204;
International patent application publication no. WO 94/19351;
Japanese patent application 5-222000;
European Journal Of Pharmacology, 251, (1994), 1; and
International patent application publication no. WO 94/22855.
α-antagonists and salts thereof, in addition to those specifically identified above, have been widely disclosed in the patent literature, including U.S. patents 4,188,390, 4,026,894, 3,511,836, 4,315,007, 3,527,761, 3,997,666, 2,503,059, 4,703,063, 3,381,009, 4.252,721, and 2,599,000.

The α-antagonism of a compound, and therefore its suitability for use in the present invention, can be determined using a number of conventional assays *in vitro.* Suitable assays include those disclosed in U. S. patent 5,599,810 which employ rabbit aorta to determine α₁-adrenoceptor antagonist activity and guinea pig left atrium to determine α₂, and in U.S. 5,340,814 which employ rat brain cortex membranes to determine both α₁ and α₂ antagonist activity.

The cGMP PDE inhibition of a compound can also be determined by standard assays known to the art, for example as disclosed in US 5,250,534, incorporated herein by reference. Compounds which are selective inhibitors of cGMP PDE relative to cAMP PDE are preferred, and determination of such compounds is also taught in US 5,250, 534. Particularly preferred are compounds which selectively inhibit the PDEv isoenzyme, as disclosed in the aforementioned PCT/EP94/01580, published as WO 94/28902.

As disclosed above, individual compounds of the combinations useful in this invention will generally be administered separately, each by its own customary and known route, and in certain cases the routes of administration may be different. In a preferred embodiment, administration will generally be timed so that both the α₁₋antagonist and the cGMP PDEᵥ inhibitor both coincide, or nearly coincide, in reaching their maximum pharmacokinetic effect. The routes of administration can be any of those known to the art such as oral, parenteral via local injection intracavernosally or intraurethrally, or transdermal as by applying the active component in a gel or other such formulation topically to the penis. Each component can be formulated as known in the art, usually together with a pharmaceutically acceptable carrier or diluent, for example as a tablet, capsule, lozenge, troche, elixir, solution, or suspension for oral administration, in a suitable injectable vehicle for parenteral administration, or as a lotion, ointment or cream for topical application. In a preferred embodiment, the cGMP PDEᵥ inhibitor and the α₁-antagonist are each co- administered orally, together or separately.

The exact dose of each component administered will, of course, differ depending on the specific components prescribed, on the subject being treated, on the severity of the impotence or of the female sexual dysfunction, on the manner of administration and on the judgment of the prescribing physician. Thus, because of patient-to-patient variability, the dosages given below are a guideline and the physician may adjust doses of the compounds to achieve the treatment that the physician considers appropriate for the patient, male or female. In considering the degree of treatment desired, the physician must balance a variety of factors such as the age of the patient and the presence of other diseases or conditions (e.g., cardiovascular disease). In general, the cGMP PDEᵥ inhibitor will be administered in a range of from 0.5 to 200 mg per day, preferably 10 to 125 mg per day, more preferably 25-100 mg per day. The α₁-antagonist will generally be administered in an amount of from 0.01 mg to 50 mg per day, preferably from 0.5 to 10 mg per day.

As previously disclosed, the combination of cGMP PDEᵥ inhibitor and α₁₋adrenoceptor antagonist can be administered as a composition. Thus, the compounds of this invention can be administered together in any conventional oral, parenteral, rectal or transdermal dosage form, usually also together with a pharmaceutically acceptable carrier or diluent.

For oral administration a pharmaceutical composition can take the form of solutions, suspensions, tablets, pills, capsules, powders, and the like. Tablets containing various excipients such as sodium citrate, calcium carbonate and calcium phosphate are employed along with various disintegrants such as starch and preferably potato or tapioca starch and certain complex silicates, together with binding agents such as polyvinyl pyrrol idone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well- known to those skilled in the art.

For purposes of transdermal (e.g.,topical) administration, dilute sterile, aqueous or partially aqueous solutions (usually in about 0.1 % to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples of methods of preparing pharmaceutical compositions, see Remington's Pharmaceutical Sciences, Mack Publishing Company, Easter, Pa., 15^{th} Edition (1975).

A combination of an α₁-antagonist and a cGMP PDEᵥ inhibitor can be tested *in vivo* in either a beagle dog or monkey model. The following description is with respect to monkeys, but those skilled in the art will easily recognize that the test applies equally and can be adapted to beagle dogs.

Mature adult male monkeys, typically either *Cercopithecus aethiops* (green monkey) or *Macaca fasciculata* (cynomologous) having a weight range of 4 to 8 kg are used. Animals are anesthetized with diazepam (2.5 mg), ketamine chloride (20 µg/kg i.m. supplemented as appropriate) and given the appropriate compound(s) dissolved in saline intracavernosally (0.3 ml). Animals are placed supine, the penis stretched out, and a rubber band placed around the root of the base as a tourniquet kept in place for three minutes after the injection. The solution is injected through a 27G needle into one of the corpus cavernosa and 5, 10, 25, 30, 60, and 180 minutes later tumescence (increase in volume) and rigidity of the penis is estimated visually and by palpitation. To determine the threshold effect using the injectable solution a series of animals are used covering an appropriate dose range for the test compound or compounds. The threshold effect is determined for the test compound or compounds.

The combination of an α₁-antagonist and cGMP PDEᵥ inhibitor can also be tested clinically, typically orally, in humans as well as in an animal model. Each component is administered singly at different times to a population of male patients, each component being administered in an amount which produces little or no response, typically less than a 50% response, as measured by the Rigiscan Clinical Evaluation parameters (see Kaneko et al., J. Urol. 136, 1026-1029 (1986); and Ogric et al., J. Urol., 154, 1356-1359 (1995)) of rigidity and tumescence, in conjunction with the International Index of Erectile Function (IIEF) questionnaire which evaluates patient and partner satisfaction. By administering each component singly, it is meant that one component is administered, followed at a later time by the second component after having allowed an appropriate time for washout of the first component. After the washout period for each component administered singly, the components are co-administered in a manner such that both components co-operate pharmacokinetically, preferably such that the peak pharmacokinetic effect due to each coincides. Co-administration is evaluated according to the regiscan parameters mentioned above and by IIEF questionnaires, thereby providing a basis for comparison of the effects of co-administration with that for each single administration.

## Claims

1. The use of:
(1) a compound selected from α₁-adrenergic antagonists, and
(2) a compound which is a cGMP PDEᵥ inhibitor; for the manufacture of a medicament for the treatment of impotence.

2. The use according to claim 1, wherein said cGMP PDEᵥ inhibitor is sildenafil or a pharmaceutically acceptable salt thereof.

3. The use according to claim 2, wherein said salt is the citrate salt.

4. The use according to claim 1, wherein said cGMP PDE inhibitor has the structure

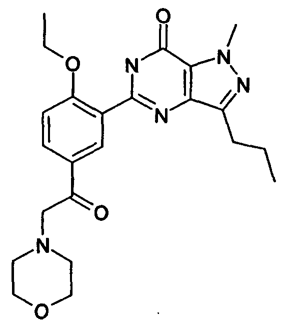

5. The use according to claim 1, wherein said cGMP PDE inhibitor has the structure 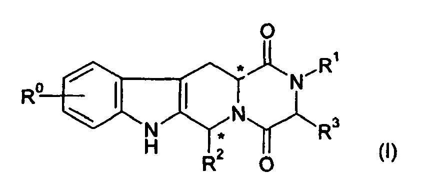 and salts and solvates thereof, in which:
R⁰ represents hydrogen, halogen or C₁₋₆alkyl,
R¹ represents hydrogen, C₁₋₆alkyl, C₂₋₆alkenyl, C₂₋₆alkynyl, haloC₁₋₆alkyl, C₃₋₈cycloalkyl, C₃₋₈cycloalkylC₁₋₃alkyl, arylC₁₋₃alkyl or heteroarylC₁₋₃alkyl;
R² represents an optionally substituted monocyclic aromatic ring selected from benzene, thiophene, furan and pyridine or an optionally substituted bicyclic ring 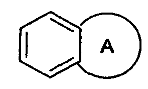 attached to the rest of the molecule via one of the benzene ring carbon atoms and wherein the fused ring A is a 5- or 6- membered ring which may be saturated or partially or fully unsaturated and comprises carbon atoms and optionally one or two heteroatoms selected from oxygen, sulphur and nitrogen; and
R³ represents hydrogen or C₁₋₃alkyl, or R¹ and R³ together represent a 3- or 4-membered alkyl or alkenyl chain.

6. The use according to claim 1, wherein said cGMP PDE inhibitor has the structure 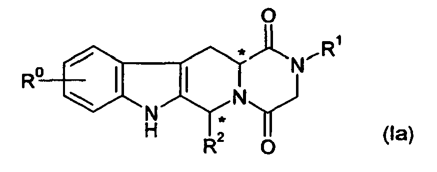
and salts and solvates thereof, in which:
R⁰ represents hydrogen, halogen or C₁₋₆alkyl,
R¹ represents hydrogen, C₁₋₆alkyl, haloC₁₋₆alkyl, C₃₋₈cycloalkyl, C₃₋₈cycloalkyl-C₁₋₃alkyl, arylC₁₋₃alkyl or heteroarylC₁₋₃alkyl;
R² represents an optionally substituted monocyclic aromatic ring selected from benzene, thiophene, furan and pyridine or an optionally substituted bicyclic ring 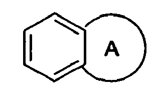 attached to the rest of the molecule via one of the benzene ring carbon atoms and wherein the fused ring A is a 5- or 6- membered ring which may be saturated or partially or fully unsaturated and comprises carbon atoms and optionally one or two heteroatoms selected from oxygen, sulphur and nitrogen.

7. The use according to claim 1, wherein said α₁-adrenergic antagonist is selected from doxazosin, terazosin, abanoquil, prazosin, alfuzosin, indoramin, naftopidil, tamsulosin, dapiprazole, and pharmaceutically acceptable salts thereof.

8. The use according to claim 1, wherein said α₁-adrenergic antagonist is selected from doxazosin, terazosin, abanoquil, prazosin and pharmaceutically acceptable salts thereof.

9. The use according to claim 8, wherein said α₁-adrenergic antagonist is doxazosin, abanoquil, or a pharmaceutically acceptable salt of either.

10. The use according to claim 9, wherein said α₁-antagonist is doxazosin mesylate or abanoquil mesylate.

11. The use according to claim 2, wherein said α₁-adrenergic antagonist is doxazosin, abanoquil, or a pharmaceutically acceptable salt of either.

12. The use according to claim 3, wherein said α₁-antagonist is doxazosin mesylate.

13. The use according to claim 3, wherein said α₁-antagonist is abanoquil mesylate.

14. The use according to claim 1, wherein the medicament is oral.

15. The use according to claim 1, wherein (1) and (2) are co-formulated.

16. The use according to claim 1, wherein (1) and (2) are administered separately.

17. A composition, comprising:
(1) a first compound selected from α₁-adrenoceptor antagonists;
(2) a second compound which is a cGMP PDEᵥ inhibitor; and
(3) a pharmaceutically acceptable carrier.

18. A composition according to claim 17, wherein said cGMP PDEᵥ inhibitor is as defined in any one of claims 2 to 6.

19. A composition according to claim 17, wherein said α₁-adrenoceptor antagonist is as defined in any one of claims 7 to 12.

20. A kit comprising:
(1) a therapeutically effective amount of a first composition comprising a compound selected from α₁-adrenergic antagonists, plus a pharmaceutically acceptable carrier or diluent, in a first dosage form;
(2) a therapeutically effective amount of a first composition comprising a cGMP PDEᵥ inhibitor, plus a pharmaceutically acceptable carrier or diluent, in a second dosage form; and
(3) container means for containing said first and second dosage forms.

21. A kit according to claim 20, wherein said cGMP elevator is as defined in any one of claims 2 to 6.

22. A kit according to claim 20, wherein said α₁-adrenoceptor antagonist is as defined in any one of claims 7 to 12.

23. A kit as defined in claim 20, wherein (1) is an α₁-adrenergic antagonist selected from doxazosin, terazosin, abanoquil, prazosin, and pharmaceutically acceptable salts thereof; and (2) is sildenafil or a pharmaceutically acceptable salt thereof.

24. A kit as defined in claim 23, wherein said α₁-adrenergic antagonist is doxazosin or a pharmaceutically acceptable salt thereof.

25. A kit as defined in claim 23, wherein said sildenafil salt is the citrate.

26. A kit as defined in claim 20, wherein (1) and (2) are each administered orally.

## Patentansprüche

1. Verwendung von
(1) einer Verbindung, die aus α₁-Adrenorezeptorantagonisten ausgewählt ist, und
(2) einer Verbindung, die ein cGMP-PDEᵥ-Inhibitor ist, zur Herstellung eines Medikaments zur Behandlung von Impotenz.

2. Verwendung nach Anspruch 1, wobei der cGMP-PDEᵥ₋Inhibitor Sildenafil oder ein pharmazeutisch akzeptables Salz desselben ist.

3. Verwendung nach Anspruch 2, wobei das Salz das Citratsalz ist.

4. Verwendung nach Anspruch 1, wobei der cGMP-PDE-Inhibitor die Struktur 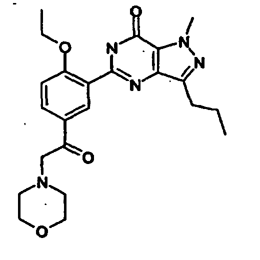 aufweist.

5. Verwendung nach Anspruch 1, wobei der cGMP-PDE-Inhibitor die Struktur 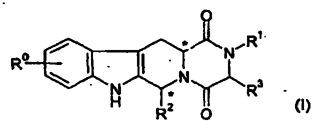 aufweist und Salze und Solvate hiervon, worin
R⁰ für Wasserstoff, Halogen oder C₁₋₆-Alkyl steht;
R¹ für Wasserstoff, C₁₋₆-Alkyl, C₂₋₆-Alkenyl, C₂₋₆-
Alkinyl, Halogen-C₁₋₆-alkyl, C₃₋₈-Cycloalkyl, C₃₋₈₋Cycloalkyl-C₁₋₃-alkyl, Aryl-C₁₋₃-alkyl oder Heteroaryl-C₁₋₃-alkyl steht;
R² für einen optional substituierten monocyclischen aromatischen Ring, der aus Benzol, Thiophen, Furan und Pyridin ausgewählt ist, oder einen optional substituierten bicyclischen Ring 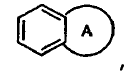 der über eines der Benzolringkohlenstoffatome an den Rest des Moleküls gebunden ist und wobei der ankondensierte Ring A ein 5- oder 6-gliedriger Ring ist, der gesättigt oder partiell oder vollständig ungesättigt sein kann und Kohlenstoffatome und optional ein oder zwei Heteroatome, die aus Sauerstoff, Schwefel und Stickstoff ausgewählt sind, umfasst, steht; und
R³ für Wasserstoff oder C₁₋₃-Alkyl steht, oder R¹ und R³ zusammen für eine 3- oder 4-gliedrige Alkyl- oder Alkenylkette stehen.

6. Verwendung nach Anspruch 1, wobei der cGMP-PDE-Inhibitor die Struktur 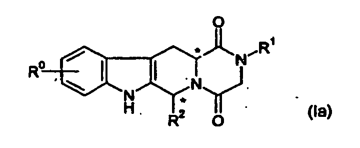 aufweist und Salze und Solvate hiervon, worin
R⁰ für Wasserstoff, Halogen oder C₁₋₆-Alkyl steht;
R¹ für Wasserstoff, C₁₋₆-Alkyl, Halogen-C₁₋₆-alkyl, C₃₋₈₋Cycloalkyl, C₃₋₈-Cycloalkyl-C₁₋₃-alkyl, Aryl-C₁₋₃-alkyl oder Heteroaryl-C₁₋₃-alkyl steht;
R² für einen optional substituierten monocyclischen aromatischen Ring, der aus Benzol, Thiophen, Furan und Pyridin ausgewählt ist, oder einen optional substituierten bicyclischen Ring 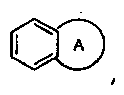 der über eines der Benzolringkohlenstoffatome an den Rest des Moleküls gebunden ist und wobei der ankondensierte Ring A ein 5- oder 6-gliedriger Ring ist, der gesättigt oder partiell oder vollständig ungesättigt sein kann und Kohlenstoffatome und optional ein oder zwei Heteroatome, die aus Sauerstoff, Schwefel und Stickstoff ausgewählt sind, umfasst, steht.

7. Verwendung nach Anspruch 1, wobei der α₁-Adrenorezeptorantagonist aus Doxazosin, Terazosin, Abanoquil, Prazosin, Alfuzosin, Indoramin, Naftopidil, Tamsulosin, Dapiprazol und pharmazeutisch akzeptablen Salzen hiervon ausgewählt ist.

8. Verwendung nach Anspruch 1, wobei der α₁-Adrenorezeptorantagonist aus Doxazosin, Terazosin, Abanoquil, Prazosin und pharmazeutisch akzeptablen Salzen hiervon ausgewählt ist.

9. Verwendung nach Anspruch 8, wobei der α₁-Adrenorezeptorantagonist Doxazosin, Abanoquil oder ein pharmazeutisch akzeptables Salz von einem der beiden ist.

10. Verwendung nach Anspruch 9, wobei der α₁-Antagonist Doxazosinmesylat oder Abanoquilmesylat ist.

11. Verwendung nach Anspruch 2, wobei der α₁-Adrenorezeptorantagonist Doxazosin, Abanoquil oder ein pharmazeutisch akzeptables Salz von einem der beiden ist.

12. Verwendung nach Anspruch 3, wobei der α₁-Antagonist Doxazosinmesylat ist.

13. Verwendung nach Anspruch 3, wobei der α₁-Antagonist Abanoquilmesylat ist.

14. Verwendung nach Anspruch 1, wobei das Medikament oral ist.

15. Verwendung nach Anspruch 1, wobei (1) und (2) coformuliert sind.

16. Verwendung nach Anspruch 1, wobei (1) und (2) getrennt verabreicht werden.

17. Zusammensetzung, die umfasst:
(1) eine erste Verbindung, die aus α₁-Adrenorezeptorantagonisten ausgewählt ist,
(2) eine zweite Verbindung, die ein cGMP-PDEᵥ₋Inhibitor ist, und
(3) einen pharmazeutisch akzeptablen Träger.

18. Zusammensetzung nach Anspruch 17, wobei der cGMP-PDEᵥ₋Inhibitor wie in einem der Ansprüche 2 bis 6 definiert ist.

19. Zusammensetzung nach Anspruch 17, wobei der α₁₋Adrenorezeptorantagonist wie in einem der Ansprüche 7 bis 12 definiert ist.

20. Kit, das umfasst:
(1) eine therapeutisch wirksame Menge einer ersten Zusammensetzung, die eine Verbindung, die aus α₁₋Adrenorezeptorantagonisten ausgewählt ist, plus einen pharmazeutisch akzeptablen Träger oder ein pharmazeutisch akzeptables Verdünnungsmittel in einer ersten Dosierungsform umfasst,
(2) eine therapeutisch wirksame Menge einer ersten Zusammensetzung, die einen cGMP-PDEᵥ-Inhibitor plus einen pharmazeutisch akzeptablen Träger oder ein pharmazeutisch akzeptables Verdünnungsmittel in einer zweiten Dosierungsform umfasst, und
(3) Behältermittel zur Aufnahme der ersten und zweiten Dosierungsform.

21. Kit nach Anspruch 20, wobei das cGMP-Erhöhungsmittel wie in einem der Ansprüche 2 bis 6 definiert ist.

22. Kit nach Anspruch 20, wobei der α₁-Adrenorezeptorantagonist wie in einem der Ansprüche 7 bis 12 definiert ist.

23. Kit gemäß der Definition in Anspruch 20, wobei (1) ein α₁-Adrenorezeptorantagonist ist, der aus Doxazosin, Terazosin, Abanoquil, Prazosin und pharmazeutisch akzeptablen Salzen derselben ausgewählt ist; und (2) Sildenafil oder ein pharmazeutisch akzeptables Salz desselben ist.

24. Kit gemäß der Definition in Anspruch 23, wobei der α₁₋Adrenorezeptorantagonist Doxazosin oder ein pharmazeutisch akzeptables Salz desselben ist.

25. Kit gemäß der Definition in Anspruch 23, wobei das Sildenafilsalz das Citrat ist.

26. Kit gemäß der Definition in Anspruch 20, wobei (1) und (2) jeweils oral verabreicht werden.

## Revendications

1. Utilisation :
(1) d'un composé choisi parmi des antagonistes α₁-adrénergiques, et
(2) d'un composé qui est un inhibiteur de GMPc-PDEᵥ ;
pour la production d'un médicament destiné au traitement de l'impuissance.

2. Utilisation suivant la revendication 1, dans laquelle ledit inhibiteur de GMPc-PDEᵥ est le sildénafil ou un de ses sels pharmaceutiquement acceptables.

3. Utilisation suivant la revendication 2, dans laquelle ledit sel est le citrate.

4. Utilisation suivant la revendication 1, dans laquelle ledit inhibiteur de GMPc-PDE a la structure

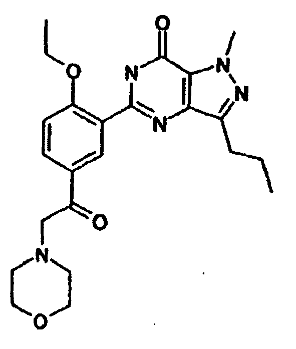

5. Utilisation suivant la revendication 1, dans laquelle ledit inhibiteur de GMPc-PDE a la structure 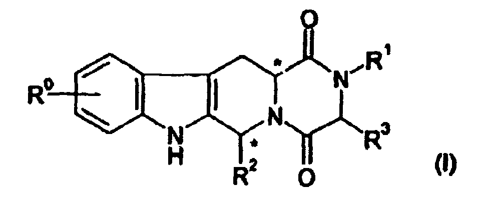 et comprend ses sels et produits de solvatation, formule dans laquelle :
R⁰ représente un atome d'hydrogène, un atome d'halogène ou un groupe alkyle en C₁ à C₆,
R¹ représente un atome d'hydrogène, un groupe alkyle en C₁ à C₆, alcényle en C₂ à C₆, alcynyle en C₂ à C₆, halogénalkyle en C₁ à C₆, cycloalkyle en C₃ à C₈, (cycloalkyle en C₃ à C₈) (alkyle en C₁ à C₃), aryl(alkyle en C₁ à C₃) ou hétéroaryl (alkyle en C₁ à C₃),
R² représente un noyau aromatique monocyclique, facultativement substitué, choisi entre des noyaux benzène, thiophène, furanne et pyridine ou un noyau bicyclique, facultativement substitué 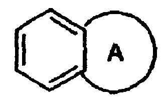 fixé au reste de la molécule par un des atomes de carbone du noyau benzénique et dans lequel le noyau condensé A est un noyau penta- ou hexagonal qui peut être saturé ou bien partiellement ou totalement insaturé et qui comprend des atomes de carbone et éventuellement un ou deux hétéroatomes choisis entre l'oxygène, le soufre et l'azote ; et
R³ représente un atome d'hydrogène ou un groupe alkyle en C₁ à C₃, ou bien R¹ et R³, conjointement, représentent une chaîne alkyle ou alcényle de 3 ou 4 membres.

6. Utilisation suivant la revendication 1, dans laquelle ledit inhibiteur de GMPc-PDE a la structure 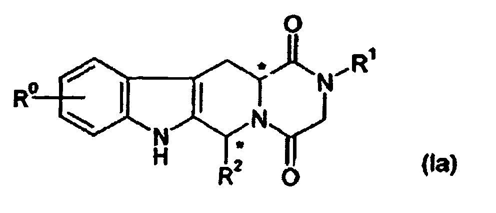 et comprend ses sels et produits de solvatation, formule dans laquelle :
R⁰ représente un atome d'hydrogène, un atome d'halogène ou un groupe alkyle en C₁ à C₆,
R¹ représente un atome d'hydrogène, un groupe alkyle en C₁ à C₆, halogénalkyle en C₁ à C₆, cycloalkyle en C₃ à C₈, (cycloalkyle en C₃ à C₈) (alkyle en C₁ à C₃), aryl(alkyle en C₁ à C₃) ou hétéroaryl (alkyle en C₁ à C₃),
R² représente un noyau aromatique monocyclique facultativement substitué, choisi entre les noyaux benzène, thiophène, furanne et pyridine ou un noyau bicyclique facultativement substitué 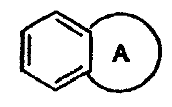 fixé au reste de la molécule par un des atomes de carbone du noyau benzénique et dans lequel le noyau condensé A est un noyau penta- ou hexagonal qui peut être saturé ou bien partiellement ou totalement insaturé et qui comprend des atomes de carbone et éventuellement un ou deux hétéroatomes choisis entre l'oxygène, le soufre et l'azote.

7. Utilisation suivant la revendication 1, dans laquelle ledit antagoniste α₁-adrénergique est choisi entre la doxazosine, la térazosine, l'abanoquil, la prazosine, l'alfuzosine, l'indoramine, le naftopidil, la tamsulosine, le dapiprazole et leurs sels pharmaceutiquement acceptables.

8. Utilisation suivant la revendication 1, dans laquelle ledit antagoniste α₁-adrénergique est choisi entre la doxazosine, la térazosine, l'abanoquil, la prazosine et leurs sels pharmaceutiquement acceptables.

9. Utilisation suivant la revendication 8, dans laquelle ledit antagoniste α₁-adrénergique est la doxazosine, l'abanoquil ou un de leurs sels pharmaceutiquement acceptables.

10. Utilisation suivant la revendication 9, dans laquelle ledit antagoniste α₁ est le mésylate de doxazosine ou le mésylate d'abanoquil.

11. Utilisation suivant la revendication 2, dans laquelle ledit antagoniste α₁-adrénergique est la doxazosine, l'abanoquil ou un de leurs sels pharmaceutiquement acceptables.

12. Utilisation suivant la revendication 3, dans laquelle ledit antagoniste α₁-adrénergique est le mésylate de doxazosine.

13. Utilisation suivant la revendication 3, dans laquelle ledit antagoniste α₁ est le mésylate d'abanoquil.

14. Utilisation suivant la revendication 1, dans laquelle le médicament est un médicament oral.

15. Utilisation suivant la revendication 1, dans laquelle les constituants (1) et (2) sont coformulés.

16. Utilisation suivant la revendication 1, dans laquelle les constituants (1) et (2) sont administrés séparément.

17. Composition comprenant :
(1) un premier composé choisi parmi des antagonistes des adrénorécepteurs α₁ ;
(2) un second composé qui est un inhibiteur de GMPc-PDEᵥ ; et
(3) un support pharmaceutiquement acceptable.

18. Composition suivant la revendication 17, dans laquelle ledit inhibiteur de GMPc-PDEᵥ est tel que défini dans l'une quelconque des revendications 2 à 6.

19. Composition suivant la revendication 17, dans laquelle ledit antagoniste des adrénorécepteurs α₁ est tel que défini dans l'une quelconque des revendications 7 à 12.

20. Kit comprenant :
(1) une première quantité thérapeutiquement efficace d'une première composition comprenant un composé choisi parmi des antagonistes α₁-adrénergiques, plus un support ou diluant pharmaceutiquement acceptable, dans une première forme posologique ;
(2) une quantité thérapeutiquement efficace d'une première composition comprenant un inhibiteur de GMPc-PDEᵥ, plus un support ou diluant pharmaceutiquement acceptable, dans une seconde forme posologique ; et
(3) des moyens servant de récipient pour contenir lesdites première et seconde formes posologiques.

21. Kit suivant la revendication 20, dans lequel ledit élévateur de GMPc est tel que défini dans l'une quelconque des revendications 2 à 6.

22. Kit suivant la revendication 20, dans lequel ledit antagoniste des adrénorécepteurs α₁ est tel que défini dans l'une quelconque des revendications 7 à 12.

23. Kit suivant la revendication 20, dans lequel le constituant (1) est un antagoniste α₁-adrénergique choisi entre la doxazosine, la térazosine, l'abanoquil, la prazosine et leurs sels pharmaceutiquement acceptables, et le constituant (2) est le sildénafil ou un de ses sels pharmaceutiquement acceptables.

24. Kit suivant la revendication 23, dans lequel ledit antagoniste α₁-adrénergique est la doxazosine ou un de ses sels pharmaceutiquement acceptables.

25. Kit suivant la revendication 23, dans lequel ledit sel de sildénafil est le citrate.

26. Kit suivant la revendication 20, dans lequel les constituants (1) et (2) sont chacun administrés par voie orale.
